# EUROPEAN PATENT SPECIFICATION

(11) **EP 2 618 885 B1**
(45) Date of publication and mention of the grant of the patent: **27.11.2019**
(21) Application number: 11826489.4
(22) Date of filing: 21.09.2011
(51) Int. Cl.: A61M 25/06, A61M 5/32

(54) **CATHETER ASSEMBLY WITH IMPROVED SAFETY MEANS**
KATHETERBAUGRUPPE MIT VERBESSERTER SICHERHEITSFUNKTION
ENSEMBLE CATHÉTER À MOYEN DE SÉCURITÉ AMÉLIORÉ

(30) Priority: 21.09.2010 IN DE22532010
(43) Date of publication of application: 31.07.2013
(73) Proprietor: Poly Medicure Limited, Faridabad, Haryana 121004 (IN)
(72) Inventor: BAID, Rishi, New Delhi 110 048 (IN)
(74) Representative: Thum, Bernhard
(86) International application number: PCT/IB2011/054137
(87) International publication number: WO 2012/038900

(56) References cited:
- EP-A1- 1 250 943
- EP-A1- 2 127 692
- EP-A1- 2 127 692
- WO-A1-2011/154767
- WO-A1-2011/154767
- WO-A2-01/93940
- WO-A2-2010/061405
- WO-A2-2010/061405
- US-B1- 6 213 978

## Description

### FIELD OF THE DISCLOSURE

The present disclosure relates in general to the intravenous catheter assembly. More particularly, the present disclosure relates to a catheter assembly with improved safety means that automatically covers the sharp tip of the needle after withdrawal of the needle from the catheter and catheter hub.

### BACKGROUND OF THE INVENTION

A catheter assembly of the above-kind is generally known and typically used to facilitate insertion and placement of a catheter or another medical device into a patient's vasculature.

Due to the increasing incidence of blood-borne pathogens such as human immunodeficiency virus (HIV), hepatitis B virus (HBV) and hepatitis C virus (HCV) there is a constant need to protect clinicians or other healthcare workers or personnel handling catheter assemblyes from accidental contact with the sharp needle tip after withdrawal of the needle from the catheter and catheter hub.

EP 2 127 692 A1 dicloses a catheter assembly comprising a needle safety device slidably arranged on the needle, wherein the needle safety device is retained in the chamber of the introducer hub when the needle extends through the introducer hub and the introducer sheath and is removable from the introducer hub once the needle tip is received in the needle safety device upon withdrawal of the needle from the introducer sheath.

WO 2011/154767 A1 discloses a catheter apparatus comprising a needle guard slidably arranged on the needle, wherein the needle guard is retained in the chamber of the catheter hub when the needle extends through the catheter hub and the catheter, and wherein the needle guard is removable from the catheter hub once the needle tip is received in the needle guard upon withdrawal of the needle from the catheter.

EP 1 250 943 A1 discloses a catheter introducer assembly comprising a safety spring clip, which moves into position to block the needle tip as a direct consequence of the withdrawal of the needle from the catheter bore.

### SUMMARY OF THE INVENTION

A catheter assembly according to the present invention is defined in claim 1. Preferred features are defined in the dependent claims.

### SUMMARY OF THE DISCLOSURE

In an aspect useful for understanding the disclosure, an intravenous catheter assembly comprises a catheter, a catheter hub having a distal section and a proximal section, wherein the distal section is joined to the catheter and the proximal section defines a chamber, a needle extending through the catheter hub and the catheter and defining an axial direction, wherein the needle has opposite proximal and distal ends, the distal end forming a tip, wherein the distal end of the said needle has a different dimension (bulge or crimp) proximal to the said needle tip, a needle hub attached to the proximal end of the needle; and a needle guard slidably arranged on the needle for protecting the said needle tip having a base portion having a bore extending in an axial direction through the bore for receiving a needle; and two opposing jaws extending from the base portion generally in the axial direction and each having a head portion in the region of its free end characterized in that the said needle guard is secured partly or completely in the chamber of the catheter hub or completely outside the catheter hub by means configured inside and/or outside of the said needle guard and by means configured inside and/or outside of the said catheter hub when the needle extends through the said catheter hub and the catheter, wherein the said needle guard is removable from the catheter hub once the slot or crimp proximal to the said needle tip is received in the needle guard upon withdrawal of the needle from the said catheter and wherein the said needle guard comprising a resilient member or elastic element or tension ring surrounding the two opposing jaws integrated therewith in a region between the base portion and the head portions of the said needle guard.

The needle as described has a bulge or crimp proximal to the tip of the needle. This bulge or crimp is used as an obstruction when the needle guard is in the protective position i.e. covering the needle tip. As the needle guard slides along the needle the bulge or crimp proximal to the needle tip engages with the base of the needle guard and locks the needle guard over the tip, thereby preventing slipping.

The means configured on the inner surface of the needle guard may comprise at least one engaging and/or retaining projection and/or recess and/or a combination of a projection and a recess capable of securing needle guard partially and/or completely in the chamber of the catheter hub.

The means configured on the outer surface of the needle guard may comprise retaining and/ or engaging arms connected to the base portion of the needle guard retaining and/or engaging partially and/or completely in the chamber of the catheter hub when the needle extends through the catheter hub and the catheter, and wherein the needle guard is prompted to protect the needle tip once the bulge, hole, slot and/or depression proximal to the said needle tip is received in the needle guard upon withdrawal of the needle from the catheter.

The means configured on the inner surface of the catheter hub may comprise at least one annular projection and/or recess and/or a combination of a projection and a recess configured inside the said catheter hub to secure the needle guard partially and/or completely in the chamber of the catheter hub.

The means configured on the outer surface of the catheter hub may comprise at least one engaging and/or retaining projection and/or recess and/or a combination of a projection and a recess to engage and/or retain the needle guard partially and/or completely in the chamber of the catheter hub.

It is thus a primary object of this disclosure to provide a catheter assembly which can be manufactured at a low cost and which is easy in its operation and design also same provides reliable protection against accidental pricking by the needle once the needle has been withdrawn from the catheter and catheter hub.

### BRIEF DESCRIPTION OF THE DRAWINGS

Figs. 1A and B show side views of a catheter assembly of the disclosure prior to use;
Fig. 2 shows a perspective view of a needle, a needle hub attached thereto and a needle guard guarding a tip of the needle after use of the catheter assembly of Fig. 1;
Figs. 3A to C show perspective views (A and B) and a side view (C) of the needle guard of Fig. 2; and
Fig. 4 shows a longitudinal sectional view of a needle guard received in a catheter hub of the catheter assembly of Fig. 1;
Figs. 5A and B show a side view (A) and a perspective view (C) of an alternative needle guard; and
Figs. 6A to C show a side view (A), a perspective view (B) and a longitudinal sectional view (C) of the needle guard of Fig. 5 without tension element.
Fig. 7 shows a longitudinal sectional view of a needle guard partly received in a catheter hub of the catheter assembly of Fig. 1;
Fig. 8 shows a longitudinal sectional view of a needle guard completely outside in a catheter hub of the catheter assembly of Fig. 1;

### DETAILED DESCRIPTION OF THE INVENTION AND DRAWINGS

A reading of all figures together describes the disclosure embodying an intravenous catheter assembly 10 comprising a catheter 12, a catheter hub 14 having a distal section and a proximal section, wherein the distal section is joined to the catheter 12 and the proximal section defines a chamber, a needle 16 extending through the catheter hub 14 and the catheter 12 and defining an axial direction, wherein the needle 16 has opposite proximal and distal ends, the distal end forming a tip 18, wherein the distal end of the said needle has a different dimension (crimp or bulge) proximal to the said needle tip 18, a needle hub 20 attached to the proximal end of the needle 16; and a needle guard 26 slidably arranged on the needle 16 for covering the said needle tip 18 having a base portion 28 having a bore 52 extending in an axial direction through the bore 52 for receiving a needle 16; and two opposing jaws (30, 32) extending from the base portion 28 generally in the axial direction and each having a head portion in the region of its free end wherein the said needle guard 26 is secured partly or completely (not part of the invention) in the chamber of the catheter hub 14 or completely outside the catheter hub by means 52 configured inside and/or outside of the said needle guard 26 and by means 80 configured inside and/or outside of the said catheter hub 14 when the needle 16 extends through the said catheter hub 14 and the catheter 12, wherein the said needle guard 26 is removable from the catheter hub 14 once the crimp or bulge proximal to the said needle tip 18 is received in the needle guard 26 upon withdrawal of the needle 16 from the said catheter 12 and wherein the said needle guard 26 comprising a resilient member 48 or elastic element 48 or tension ring 48 surrounding the two opposing jaws (30, 32) integrated therewith in a region between the base portion and the head portions of the said needle guard.

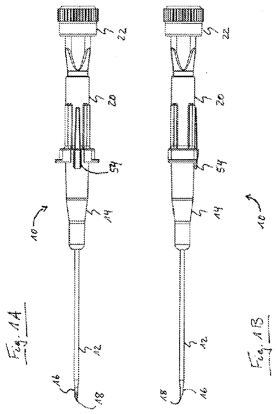

Fig. 1 shows a catheter assembly 10 in accordance with the disclosure prior to use.

The catheter assembly 10 includes a tube-like catheter 12 having distal and proximal ends, and a catheter hub 14 attached to the catheter 12 at the proximal end of the catheter 12.

The catheter assembly 10 further comprises a needle 16 extending through the catheter 12 and catheter hub 14 and defining an axial direction. The needle 16 has distal and proximal ends, wherein a sharp needle tip 18 is formed at the distal end of the needle 16, which protrudes from the catheter 12 at the distal end thereof.

A needle hub 20 is attached to the needle 16 at the proximal end of the needle 16. A port member 22 is mounted to the needle hub 20 at a proximal side of the needle hub 20, which makes it possible to connect, for example, a PVC tube to the catheter assembly 10 for collecting blood from a patient or delivering a fluid to be administered to a patient.

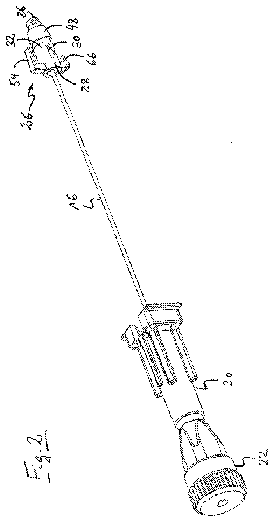

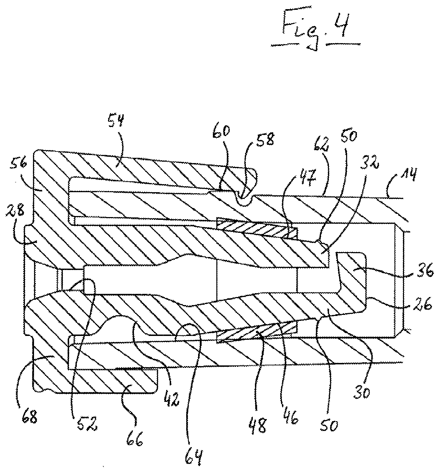

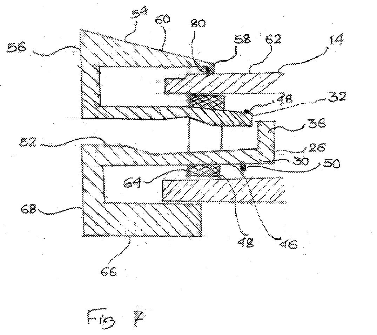

The catheter hub 14 defines an inner chamber (Fig. 4 & 7) in which a needle guard 26 is fully or partly seated. In Fig. 1 only a retaining arm 54 of the needle guard 26 can be seen. The needle guard 26 is slidably arranged on the needle 16, such that it moves along the needle 16 when the needle 16 is withdrawn from the catheter 12. The purpose of the needle guard 26 is to cover the needle tip 18 after the needle 16 has been used in order to prevent accidental pricking by the needle 16 of a person handling the catheter assembly 10, as can be seen from Fig. 2.

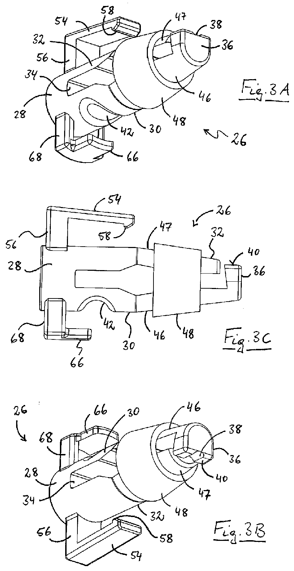

As shown in Fig. 3, the needle guard 26 includes a generally cylindrical base portion 28 at a proximal end of the needle guard 26. First and second arms 30, 32 extend from a distal face 34 of the base portion 28 generally in the axial direction. The first arm 30 is longer than the second arm 32.

A transverse wall 36 is arranged in a distal region of the first arm 30. The dimension of the transverse wall 36 as seen in a radial direction is selected such that the transverse wall 36 overlaps with the second arm 32 when the needle tip 18 is received in the needle guard 26, i.e. between the first and second arms 30, 32, such that the needle tip 18 is blocked by the transverse wall 36 from distally protruding from the needle guard 26 (Fig. 2).

Prior to use, i.e. when the needle 16 extends all the way through the needle guard 26, the transverse wall 36 is supported on the needle 16 and the first arm 30 is deflected away from the second arm 32. An axial groove 38 is provided in the transverse wall 36 on its side 40 facing the needle 16 in order to guide the transverse wall 36 on the needle 16, in particular when the needle 16 is pulled through the needle guard 26 upon withdrawal of the needle 16 from the catheter 12.

A recess 42 is provided in the outer surface of a proximal section 44 of the first arm 30 in order to increase the deflectability of the first arm 30.

Distal sections 46, 47 of the first and second arms 30, 32 have outer surfaces that are tapered towards the distal ends of the first and second arms 30, 32. The first and second arms 30, 32 are surrounded by an elastic band 48 in the region of the distal sections 46, 47. Because of the tapered outer surfaces of the distal sections 46, 47 the elastic band 48 is prevented from sliding off the distal sections 46, 47 towards the proximal end of the needle guard 26 when the first and second arms 30, 32 are spread apart against a restoring force of the elastic band 48 by the needle 16 extending all the way through the needle guard 26. In order to further define the axial position of the elastic band 48, protrusions 50 extending along the outer periphery of the first and second arms 30, 32 can be provided adjacent the elastic band 48 (Fig. 4). These protrusions 50 are not shown in Figs. 2 and 3.

As is best seen in Fig. 4 and 7, an axial bore 52 extends through the base portion 28 of the needle guard 26 for receiving the needle 16. The profile of the bore 52 is adapted to the principle outer profile of the needle 16. The needle 16 has a change in profile, in particular an enlargement (not shown), near its needle tip 18, which has an outer profile one dimension of which is larger than a maximum dimension of the profile of the bore 52. Thus, the change in profile prevents the needle guard 26 from sliding off the needle 16 when the needle tip 18 is received between the first and second arms 30, 32 and covered by the needle guard 26.

In another construction an axial bore 52 extends through the base portion 28 of the needle guard 26 for receiving the needle 16. The profile of the bore 52 is adapted to the principle outer profile of the needle 16. The needle 16 has a change in profile, in particular a crimp(not shown), near its needle tip 18, which has an outer profile one dimension of which is smaller than a maximum dimension of the profile of the bore 52. Thus, the change in profile prevents the needle guard 26 from sliding off the needle 16 when the needle tip 18 is received between the first and second arms 30, 32 and covered by the needle guard 26.

A retaining arm 54 is provided on the needle guard 26 in order to secure the needle guard 26 in the chamber of the catheter hub and, in particular, to prevent removal of the needle guard 26 from the catheter hub 14 upon withdrawal of the needle 16 before the needle tip 18 has been safely received in the needle guard 26. The retaining arm 54 is connected to the base portion 28 of the needle guard 26 via a transverse segment 56. The transverse segment 56 extends outwardly from the base portion 28 in a generally radial direction at the side of the second arm 32. The retaining arm 54 extends distally from the transverse segment 56 and is slightly tilted towards the second arm 32. Preferably, the retaining arm 45 forms an angle in the range between 0° and 10° with the axial direction, such that a clearing between the retaining arm 54 and the second arm 32 narrows towards a distal end of the retaining arm 54.

A hook-like protrusion 58 is provided in the region of the distal end of the retaining arm 54. The hook-like protrusion 58 of the retaining arm 54 engages behind a corresponding protrusion 60 and into a groove or recess 61 provided at the outer surface 62 of the catheter hub 14. Because of the engagement between the hook-like protrusion 58 of the retaining arm 54 and the combination of protrusion 60 and recess 61 formed on the catheter hub 14, the needle guard 26 is prevented from axial movement relative to the catheter hub 14 and effectively retained at the catheter hub 14 until a pulling force exerted by the needle 16 on the base portion 28 of the needle guard 26 via the change in profile of the needle 16 upon withdrawal of the needle 16 from the catheter 12 becomes great enough to disengage the retaining arm 54 from the protrusion 60 of the catheter hub 14.

It is to be noted that instead of a combination of protrusion 60 and recess 61 formed at the catheter hub 14 it is also possible to provide either a protrusion 60 or a recess 61 in the outer surface 62 of the catheter hub 14 for engagement with the hook-like protrusion 58 of the retaining arm 54.

The retaining of the needle guard 26 in the catheter hub 14 can be further improved if the elastic band 48 engages with an inner surface 64 of the catheter hub 14 in the spread-apart state of the first and second arms 30, 32. Once the needle tip 18 passes the transverse wall 36 and the first and second arms 30, 32 snap together, thereby allowing the elastic band 48 to contract, the elastic band 48 can disengage from the inner surface 64 of the catheter hub 14.

A supporting arm 66 is provided on a side of the needle guard 26 opposite from the retaining arm 54. The supporting arm 66 forms a generally right angle with a transverse segment 68 which extends from the base portion 28 in a generally radial direction. The supporting arm 66 is configured to be in contact with the outer surface 62 of the catheter hub 14 across its substantially entire length and width. The supporting arm 66 thus prevents the needle guard 26 received in the catheter hub 14 from moving in a radial direction within the chamber. The supporting arm 66 may also help to secure the needle guard 26 against axial movement with respect to the catheter hub 14, thereby adding to the retaining of the needle guard 26 in the catheter hub 14. However, the supporting arm 66 does not necessarily have to be in such a retaining relationship with the catheter hub 14.

In figures 4 & 7, the retaining arm 54 is provided on the side of the base portion 28 adjacent the second arm 32, and the supporting arm 66 is provided on the side of the base portion 28 adjacent the first arm 30. However, it is generally also possible to provide the retaining arm 54 on the side of the base portion 28 adjacent the first arm 30 and the supporting arm 66 on the side of the base portion 28 adjacent the second arm 32.

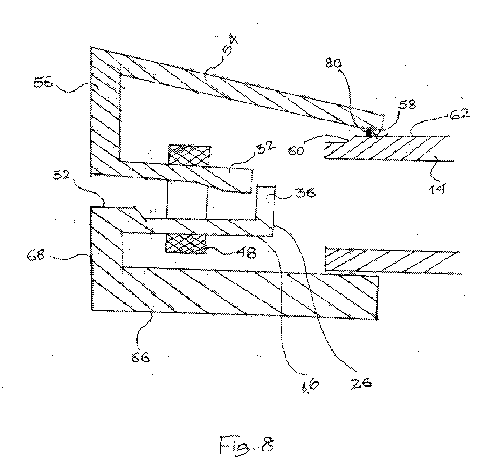

In the example in Figure 4, the needle guard 26 is completely inside the catheter hub 14 and is retained on the outside by engagement means 80 on the catheter hub 14. The engagement means 80 can be in the form of a groove as shown in Figure 4 right under hook 58 or in form of a protrusion 80 as shown in Figure 7 and 8.

As shown in Figs. 1 to 4, 7 and 8, the base portion 28, the first and second arms 30, 32, the retaining arm 54 and the supporting arm 66 are integrally formed, for example, from a plastic material by way of injection molding. However, it is to be understood that this integral design is not compulsory. Instead, one or more of the named components could be made of a material that is different from the material of the other components, and this component could be attached to the other components, for example, by gluing, welding, soldering or the like.

Although not shown in Figs. 1 to 4, 7 and 8, a stopping element may be provided in the base portion 28 or on the distal face 34 of the base portion 28, wherein the stopping element has a through-bore which is aligned with the axial bore 52 in the base portion 28 and which has a profile that is adapted to the principle outer profile of the needle 16. Preferably, such a stopping element would be made of a material of a greater hardness and/or stiffness than the material of the base portion 28, such that this stopping element withstands greater forces exerted by the change in profileof the needle 16 upon withdrawal of the needle 16 from the catheter 12, thereby more effectively preventing the change in profileof the needle 16 from passing through the base portion 28 and thus more effectively preventing the needle guard 26 from sliding off the needle 16.

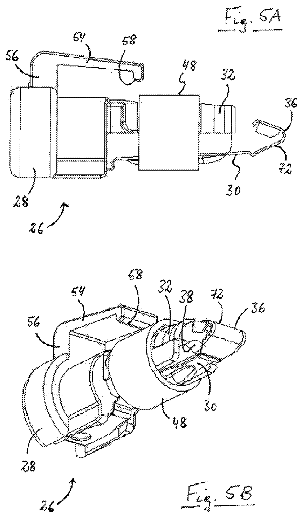

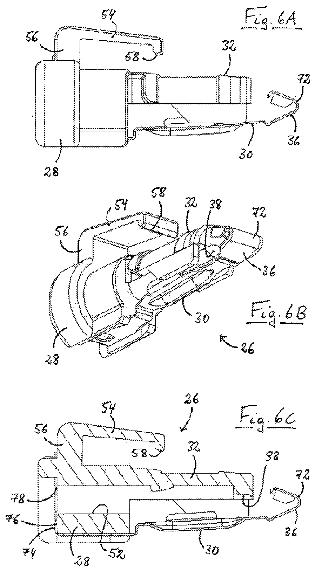

Figs. 5 and 6 show an alternative needle guard 26 that can be used in the catheter assembly 10 of the disclosure. This needle guard 26 is generally identical in function but differs from the aforedescribed needle guard 26 mainly in that it does not have any supporting arm and in that the first arm 30 is not integrally formed with the base portion 28, the second arm 32 and the retaining arm 54.

Instead, the first arm 30 is made of a strip of sheet metal 70, a distal end portion 72 of which is bent to form the transverse wall 36 and a proximal end portion 74 of which is attached, e.g. glued or welded, to a proximal face 76 of the base portion 28. As described above, distal sections 46, 47 of the first and second arms 30, 32 are surrounded by an elastic band 48 such that the first and second arms 30, 32 can be spread apart against a restoring force of the elastic band 48 (Fig. 5).

As is best seen in Fig. 6C, a through-bore 78 is provided in the proximal end portion 74 of the strip of sheet metal 70 and aligned with the axial bore 52 in the base portion 28. Theprofile of the through-bore78is adapted to the principal outer profileof the needle 16, whereas the profile of the axial bore 52 is somewhat wider. Hence, in this needle guard 26 the change of profile of the needle 16, e.g. its enlargement, can slide along the axial bore 52 and is limited in movement in the proximal direction only upon engagement with the proximal end portion 74 of the strip of sheet metal 70 at the proximal side of the base portion 28. The proximal end portion 74 of the strip of sheet metal 70 thus functions as a stopping element of the kind that has already been discussed above and helps to prevent the needle guard 26 from sliding off the needle 16.

In further contrast to the needle guard 26 shown in Figs. 1 to 4, 7 and 8, in the needle guard 26 of Figs. 5 and 6 the axial groove 38 for guiding the needle 16 upon movement relative to the needle guard 26 is not provided on the first arm 30 but on the second arm 32, as is best seen in Fig. 6B.

Figure 4 is an example of the above described disclosure and figures 7 and 8 are embodiments of the above described invention and function substantially in the same manner and for the sake of brevity repetition is being avoided.

As can be seen from the description above that base portion 28, one of first arm 30 of the said needle guard are integrally made from different material, e.g. a plastic material, and the second arm 32 is made from a second material different, such as steel, from said first material. It is well within the scope of this invention that the base portion 28, first arm 30, second arm 32 and retaining arm 54 can be made of same material. Likewise, it is well within the scope of the invention to have all the elements of needle guard as described to be unitary or in separate parts and/or different materials.

The invention is defined by the appended claims.

### Reference numeral list

10 catheter assembly
12 catheter
14 catheter hub
16 needle
18 needle tip
20 needle hub
22 port member
26 needle guard
28 base portion
30 first arm
32 second arm
34 distal face
36 transverse wall
38 axial groove
40 side
42 recess
44 proximal portion
46 distal section
47 distal section
48 elastic band
50 protrusion
52 axial bore
54 retaining arm
56 transverse segment
58 protrusion
60 protrusion
61 recess
62 outer surface
64 inner surface
66 supporting arm
68 transverse segment
70 strip of sheet metal
72 distal end portion
74 proximal end portion
76 proximal face
78 through-bore
80 Protrusion or groove on the outside of the catheter hub

## Claims

1. A catheter assembly (10) comprising:
a catheter (12);
a catheter hub (14) having a distal section and a proximal section, wherein the distal section is joined to the catheter (12) and the proximal section defines a chamber;
a needle (16) extending through the catheter hub (14) and the catheter (12) and defining an axial direction, wherein the needle (16) has opposite proximal and distal ends, the distal end forming a needle tip (18);
a needle hub (20) attached to the proximal end of the needle (16); and
a needle guard (26) comprising a generally cylindrical base portion (28) in the region of its proximal end and first and second arms (30, 32) extending in a generally axial direction from a distal side of the base portion (28), the needle guard (26) being slidably arranged on the needle (16),
wherein the needle guard (26) is partly retained in the chamber of the catheter hub (14) or the needle guard (26) is completely outside the catheter hub (14) by means of a retaining arm (54) engaging the outside of the catheter hub (14) when the needle (16) extends through the catheter hub (14) and the catheter (12), and wherein the needle guard (26) is removable from the catheter hub (14) once the needle tip (18) is received in the needle guard (26) upon withdrawal of the needle (16) from the catheter (12);
a supporting arm (66) is provided on at least one side of the needle guard (26) opposite from the retaining arm (54), wherein the supporting arm is configured to be in contact with the outer surface (62) of the catheter hub (14), **characterized in that**
the needle guard (26) is retained on the outside of the catheter hub (14) or partly retained in the chamber of the catheter hub (14) by engagement means (80) on the catheter hub (14) in the form of a protrusion.

2. A catheter assembly (10) in accordance with claim 1,
wherein a proximal end of the retaining arm (54) is connected to a base portion of the needle guard (26) by means of a transverse segment (56), in particular wherein the transverse segment (56) extends generally in a radial direction and/or from a proximal end region of the needle guard (26).

3. A catheter assembly (10) in accordance with claim 1 or 2,
wherein the retaining arm (54) extends not parallel to the axial direction and, in particular, forms an angle in the range between 0° and 10° with the axial direction, in particular such that a clearing between the retaining arm (54) and the catheter hub (14) narrows towards a distal end of the retaining arm (54).

4. A catheter assembly (10) in accordance with any one of claims 1 to 3,
wherein a hook-like protrusion (58) is provided in the region of a distal end of the retaining arm (54).

5. A catheter assembly (10) in accordance with any one of the preceding claims,
wherein the first and second arms (30, 32) are surrounded by a tension element, e.g. an elastic band (48), in a distal region of the first and second arms.

6. A catheter assembly (10) in accordance with claim 5,
wherein the first and second arms (30, 32) are spread apart by the needle (16) extending completely through the needle guard (26) such that the tension element (48) is brought into retaining engagement with an inner surface (64) of the catheter hub (14).

7. A catheter assembly (10) in accordance with any one of claims 6 or 7, wherein a transverse wall (36) is arranged in a distal region of one of the first and second arms (30, 32), wherein a groove (38) is provided in a side (40) of the transverse wall (36), the groove (38) extending substantially in the axial direction.

8. A catheter assembly (10) in accordance with any one of claims 1 to 7,
wherein the base portion (28) of the needle guard (26) has an axial bore (52) extending there through for receiving the needle (16), wherein the profile of the bore (52) is adapted to the principal outer profile of the needle (16), and the needle (16) has a change in profile near the needle tip (18), said change in profile having an outer profile one dimension of which is larger than a maximum dimension of the profile of the bore (52).

## Patentansprüche

1. Katheteranordnung (10), umfassend:
einen Katheter (12);
einen Katheteransatz (14), der einen distalen Abschnitt und einen proximalen Abschnitt aufweist, wobei der distale Abschnitt mit dem Katheter (12) verbunden ist und der proximale Abschnitt eine Kammer umgrenzt;
eine Nadel (16), die sich durch den Katheteransatz (14) und den Katheter (12) erstreckt und eine axiale Richtung bestimmt, wobei die Nadel (16) entgegengesetzte proximale und distale Enden aufweist, wobei das distale Ende eine Nadelspitze (18) ausbildet;
einen Nadelansatz (20), der am proximalen Ende der Nadel (16) befestigt ist; und
einen Nadelschutz (26), der einen im Allgemeinen zylinderförmigen Basisabschnitt (28) im Bereich seines proximalen Endes und erste und zweite Arme (30, 32) umfasst, die sich in einer im Allgemeinen axialen Richtung von einer distalen Seite des Basisabschnitts (28) erstrecken, wobei der Nadelschutz (26) verschiebbar auf der Nadel (16) angeordnet ist,
wobei der Nadelschutz (26) teilweise in der Kammer des Katheteransatzes (14) zurückgehalten wird, oder sich der Nadelschutz (26) vollständig außerhalb des Katheteransatzes (14) befindet, mittels eines Rückhaltearms (54), der außerhalb des Katheteransatzes (14) eingreift, wenn sich die Nadel (16) durch den Katheteransatz (14) und den Katheter (12) erstreckt, und wobei der Nadelschutz (26) vom Katheteransatz (14) entfernbar ist, nachdem die Nadelspitze (18) beim Zurückziehen der Nadel (16) vom Katheter (12) im Nadelschutz (26) aufgenommen wird;
ein Haltearm (66) auf wenigstens einer Seite des Nadelschutzes (26) gegenüber dem Rückhaltearm (54) vorgesehen ist, wobei der Haltearm so konfiguriert ist, dass er Kontakt mit der äußeren Oberfläche (62) des Katheteransatzes (14) hat, **dadurch gekennzeichnet, dass**
der Nadelschutz (26) auf der Außenseite des Katheteransatzes (14) mittels Eingriffsmitteln (80) auf dem Katheteransatz (14) in Form eines Vorsprungs am Katheteransatz (14) zurückgehalten wird oder teilweise zurückgehalten wird.

2. Katheteranordnung (10) nach Anspruch 1,
wobei ein proximales Ende des Rückhaltearms (54) an einem Basisabschnitt des Nadelschutzes (26) mittels eines Quersegments (56) verbunden ist, insbesondere wobei sich das Quersegment (56) im Allgemeinen in eine radiale Richtung und/oder von einem proximalen Endbereich des Nadelschutzes (26) erstreckt.

3. Katheteranordnung (10) nach Anspruch 1 oder 2, wobei sich der Rückhaltearm (54) nicht parallel zur axialen Richtung erstreckt und insbesondere einen Winkel im Bereich zwischen 0 ° und 10 ° mit der Achsenrichtung bildet, insbesondere so, dass sich ein Zwischenraum zwischen dem Rückhaltearm (54) und dem Katheteransatz (14) zu einem distalen Ende des Rückhaltearms (54) verengt.

4. Katheteranordnung (10) nach einem der Ansprüche 1 bis 3, wobei ein hakenartiger Vorsprung (58) im Bereich eines distalen Endes des Rückhaltearms (54) vorgesehen ist.

5. Katheteranordnung (10) nach einem der vorhergehenden Ansprüche, wobei der erste und zweite Arm (30, 32) von einem Spannelement, beispielsweise einem elastischen Band (48), in einem distalen Bereich des ersten und zweiten Arms umgeben sind.

6. Katheteranordnung (10) nach Anspruch 5,
wobei der erste und der zweite Arm (30, 32) von der Nadel (16) auseinander gespreizt werden, die sich vollständig durch den Nadelschutz (26) erstreckt, sodass das Spannelement (48) in rückhaltenden Eingriff mit einer Innenfläche (64) des Katheteransatzes (14) gebracht wird.

7. Katheteranordnung (10) nach einem der Ansprüche 6 oder 7, wobei eine Querwand (36) in einem distalen Bereich von einem aus dem ersten und dem zweiten Arm (30, 32) angeordnet ist, wobei eine Rille (38) in einer Seite (40) der Querwand (36) vorgesehen ist, wobei sich die Rille (38) im Wesentlichen in der axialen Richtung erstreckt.

8. Katheteranordnung (10) nach einem der Ansprüche 1 bis 7, wobei der Basisabschnitt (28) des Nadelschutzes (26) eine axiale Bohrung (52) aufweist, die sich durch denselben erstreckt um die Nadel (16) aufzunehmen, wobei das Profil der Bohrung (52) an das hauptsächliche Außenprofil der Nadel (16) angepasst ist, und die Nadel (16) nahe der Nadelspitze (18) eine Profiländerung aufweist, wobei die Profiländerung ein Außenprofil aufweist, von dem eine Abmessung größer als eine größte Abmessung des Profils der Bohrung (52) ist.

## Revendications

1. Ensemble cathéter (10) comprenant :
un cathéter (12) ;
un raccord de cathéter (14) ayant une section distale et une section proximale, la section distale étant reliée au cathéter (12) et la section proximale définissant une chambre ;
une aiguille (16) s'étendant à travers le raccord de cathéter (14) et le cathéter (12) et définissant une direction axiale, l'aiguille (16) ayant des extrémités proximale et distale opposées, l'extrémité distale formant une pointe d'aiguille (18) ;
un raccord d'aiguille (20) fixé à l'extrémité proximale de l'aiguille (16) ; et
une gaine (26) comprenant une partie de base généralement cylindrique (28) dans la région de son extrémité proximale et des premier et second bras (30, 32) s'étendant dans une direction généralement axiale depuis un côté distal de la partie de base (28), la gaine (26) étant disposée de manière coulissante sur l'aiguille (16), la gaine (26) étant partiellement retenue dans la chambre du raccord de cathéter (14) ou la gaine (26) se trouvant entièrement à l'extérieur du raccord de cathéter (14) au moyen d'un bras de retenue (54) engageant l'extérieur du raccord de cathéter (14) lorsque l'aiguille (16) s'étend à travers le raccord de cathéter (14) et le cathéter (12), et la gaine (26) étant apte à être retirée du raccord de cathéter (14) une fois que la pointe d'aiguille (18) est reçue dans la gaine (26) lors du retrait de l'aiguille (16) à partir de cathéter (12) ;
un bras de support (66) étant prévu sur au moins un côté de la gaine (26) opposé au bras de retenue (54), le bras de support étant configuré pour être en contact avec la surface externe (62) du raccord de cathéter (14),
**caractérisé par le fait que** la gaine (26) est retenue sur l'extérieur du raccord de cathéter (14) ou partiellement retenue dans la chambre du raccord de cathéter (14) par des moyens d'engagement (80) sur le raccord de cathéter (14) sous la forme d'une saillie.

2. Ensemble cathéter (10) selon la revendication 1,
dans lequel une extrémité proximale du bras de retenue (54) est reliée à une partie de base de la gaine (26) au moyen d'un segment transversal (56), en particulier le segment transversal (56) s'étendant généralement dans une direction radiale et/ou à partir d'une région d'extrémité proximale de la gaine (26).

3. Ensemble cathéter (10) selon la revendication 1 ou 2,
dans lequel le bras de retenue (54) s'étend non parallèlement à la direction axiale et, en particulier, forme un angle compris dans la plage entre 0° et 10° avec la direction axiale, en particulier de telle sorte qu'un dégagement entre le bras de retenue (54) et le raccord de cathéter (14) se rétrécit vers une extrémité distale du bras de retenue (54).

4. Ensemble cathéter (10) selon l'une quelconque des revendications 1 à 3,
dans lequel une saillie de type crochet (58) est prévue dans la région d'une extrémité distale du bras de retenue (54).

5. Ensemble cathéter (10) selon l'une quelconque des revendications précédentes,
dans lequel les premier et second bras (30, 32) sont entourés d'un élément de tension, par exemple une bande élastique (48), dans une région distale des premier et second bras.

6. Ensemble cathéter (10) selon la revendication 5,
dans lequel les premier et second bras (30, 32) sont écartés par l'aiguille (16) s'étendant entièrement à travers la gaine (26) de telle sorte que l'élément de tension (48) est amené en engagement de retenue avec une surface interne (64) du raccord de cathéter (14).

7. Ensemble cathéter (10) selon l'une quelconque des revendications 6 ou 7,
dans lequel une paroi transversale (36) est disposée dans une région distale de l'un des premier et second bras (30, 32), une rainure (38) étant prévue sur un côté (40) de la paroi transversale (36), la rainure (38) s'étendant sensiblement dans la direction axiale.

8. Ensemble cathéter (10) selon l'une quelconque des revendications 1 à 7,
dans lequel la partie de base (28) de la gaine (26) a un alésage axial (52) s'étendant à travers celle-ci pour recevoir l'aiguille (16), le profil de l'alésage (52) étant adapté au profil externe principal de l'aiguille (16), et l'aiguille (16) ayant un changement de profil près de la pointe d'aiguille (18), ledit changement de profil ayant un profil externe dont une dimension est plus grande qu'une dimension maximale du profil de l'alésage (52).
